# EUROPÄISCHE PATENTSCHRIFT

(11) **EP 2 074 100 B1**
(45) Veröffentlichungstag und Bekanntmachung des Hinweises auf die Patenterteilung: **28.08.2013**
(21) Anmeldenummer: 07818523.8
(22) Anmeldetag: 27.09.2007
(51) Int. Cl.: C07D 217/04, C07D 217/10, A61P 31/04, A61K 31/472

(54) **BIOFILM-HEMMENDE WIRKUNG SOWIE ANTI-INFEKTIVE AKTIVITÄT N,C-VERKNÜPFTER ARYLISOCHINOLINE UND DEREN VERWENDUNG**
BIOFILM-INHIBITING EFFECT AND ANTI-INFECTIVE ACTIVITY OF N,C-LINKED ARYLISOQUINOLINES AND THE USE THEREOF
EFFET D'ENTRAVEMENT DES BIOFILMS AINSI QU'ACTIVITÉ ANTI-INFECTIEUSE DES ARYLISOQUINOLÉINES N,C-LIÉES ET LEUR UTILISATION

(30) Priorität: 27.09.2006 DE 102006046922
(43) Veröffentlichungstag der Anmeldung: 01.07.2009
(73) Patentinhaber: Julius-Maximilians-Universität Würzburg, 97070 Würzburg (DE)
(72) Erfinder: BRINGMANN, Gerhard, 97074 Würzburg (DE); GULDER, Tanja, San Diego, CA 92122 (US); HENTSCHEL, Ute, 97076 Würzburg (DE); MEYER, Frank, 97199 Ochsenfurt (DE); MOLL, Heidrun, 97074 Würzburg (DE); MORSCHHÄUSER, Joachim, 97250 Erlabrunn (DE); PONTE-SUCRE DE VANEGAS, Alicia, 97072 Würzburg (DE); ZIEBUHR, Wilma, 97209 Veitshöchheim (DE); STICH, August, 97218 Gerbrunn (DE); BRUN, Reto, 4106 Therwil (CH); MÜLLER, Werner, E., G., 65203 Wiesbaden (DE); MUDOGU, Virima, Kinshasa (CG)
(74) Vertreter: Krauss, Jan
(86) Internationale Anmeldenummer: PCT/EP2007/008440
(87) Internationale Veröffentlichungsnummer: WO 2008/037482

(56) Entgegenhaltungen:
- WO-A1-03/106454
- ZHANG Y ET AL: "Superacid-promoted reactions of N-acyliminium ions: An effective route to substituted 3-oxo-1,2,3,4-tetrahydroisoquinolines and related products" SYNTHESIS 01 JUN 2006 GERMANY, Nr. 11, 1. Juni 2006 (2006-06-01), Seiten 1775-1780, XP002457920 ISSN: 0039-7881
- SZANTAY, CSABA; SZABO, LAJOS: "Chemistry of heterocyclic, pseudobasic amino carbinols. XXIX. The constitution of the bases formed from substituted 2-(2,4-dinitrophenyl)isoquinolinium salts" CHEMISCHE BERICHTE, Bd. 98, Nr. 4, 1965, Seiten 1023-1030, XP002457918
- BEKE D ET AL: "Uber eine neue isochinolin-ringschlussreaktion, VI.; A new ring closure reaction of Isoquinolines, VI" ACTA CHIMICA ACADEMIAE SCIENTIARUM HUNGARICA, BUDAPEST, HU, Bd. 35, 1963, Seiten 205-211, XP008085724 ISSN: 0001-5407
- BRINGMANN GERHARD ET AL: "Ancisheynine, the first n,c-coupled naphthylisoquinoline alkaloid: total synthesis and stereochemical analysis." ORGANIC LETTERS 16 MAR 2006, Bd. 8, Nr. 6, 16. März 2006 (2006-03-16), Seiten 1037-1040, XP002457930 ISSN: 1523-7060
- YANG L-K ET AL: "Ancisheynine, a novel naphthylisoquinolinium alkaloid from Ancistrocladus heyneanus" TETRAHEDRON LETTERS, ELSEVIER, AMSTERDAM, NL, Bd. 44, Nr. 31, 28. Juli 2003 (2003-07-28), Seiten 5827-5829, XP004435085 ISSN: 0040-4039
- PONTE-SUCRE ALICIA ET AL: "Activities of naphthylisoquinoline alkaloids and synthetic analogs against Leishmania major." ANTIMICROBIAL AGENTS AND CHEMOTHERAPY JAN 2007, Bd. 51, Nr. 1, Januar 2007 (2007-01), Seiten 188-194, XP002457919 ISSN: 0066-4804

## Beschreibung

Die vorliegende Erfindung betrifft Ansprüche 1 bis 12. Soweit nicht in den Ansprüchen enthaltern betrifft die Beschreibung weiter die anti-infektiven (anti-Candida, anti-Leishmanialen, anti-Trypanosomalen, anti-Plasmodialen) sowie Biofilm-hemmenden Aktivitäten von *N,C-*verknüpften Arylisochinolin-Derivaten und deren Verwendungen, insbesondere als bioaktive Wirkstoffe für biotechnologische und medizinische Anwendungen, besonders zur Vermeidung der Bildung von Biofilmen durch humanpathogene Bakterien, sowie das anti-infektive Potential spezifischer Vertreter der Substanzen gegen die Erreger *Plasmodien, Trypanosomen* und *Leishmanien.*

### Hintergrund der Erfindung

Laut Weltgesundheitsbericht 2002 der World Health Organization (WHO) sind die Infektionskrankheiten weltweit nach wie vor die Todesursache Nr. 1. Besonders in den Entwicklungsländern sterben jährlich Millionen Menschen an den Folgen von Malaria, Schlafkrankheit, Chagas-Krankheit, Leishmaniose, Candida-Infektionen und anderen Infektionskrankheiten. Während in den Industrieländern die klassischen Infektionskrankheiten zunächst größtenteils besiegt schienen (2002: 7% der Todesfälle in Deutschland), sind diese weltweit wieder auf dem Vormarsch: Viele der gängigen Arzneistoffe verlieren durch zunehmende Resistenzbildung der Erreger ihre Wirkung. Dazu gehören auch Gram-positive Bakterien wie Staphylokokken und Enterokokken, welche Septikämien und andere Infektionen hauptsächlich bei immunsupprimierten Patienten auslösen. Als besondere Problemkeime sind die Methicillinund Oxacillin-resistenten Staphylokokken (MRSA, ORSA), die Vancomycin-resistenten Enterokokken sowie die multiresistenten Pseudomonaden zu nennen.

Neben der immer stärkeren Resistenzbildung von mikrobiellen Erregern ist deren Bildung von Biofilmen ein großes Problem. Unter Biofilmen versteht man eine Gemeinschaft von Mikroorganismen, die sich in eine extrazelluläre Polysaccharid- oder Protein-Matrix einhüllt, wodurch die Einzelzellen befähigt werden, aneinander und/oder an Oberflächen zu haften (J. W. Costerton, Z. Lewandowski, D. E. Caldwell, D. R. Korber, H. M. Lappin-Scott, Annu. Rev. Microbiol. 1995, 49, 711-745; P. Stoodley, K. Sauer, D. G. Davies, J. W. Costerton, Annu. Rev. Microbiol. 2002, 56, 187-209). Die mikrobielle Gemeinschaft kann dabei aus einer oder auch aus mehreren Spezies bestehen. Die Organisation von Zellen in einem Biofilm führt zu einer deutlich erhöhten Widerstandsfähigkeit der gesamten Population gegenüber verschiedensten Einflüssen. Biofilme sind daher nicht als eine Ansammlung von Einzelzellen zu verstehen. Sie ähneln vielmehr in ihrer Physiologie einem multizellulären Organismus, in dem unterschiedliche Genexpression und metabolische Aktivität, Dynamik und Arbeitsteilung herrschen.

Biofilme sind in der Natur weit verbreitet. Sie finden sich bevorzugt an den Grenzflächen zwischen fester und flüssiger Phase und sind im aquatischen Milieu (Flüssen, Seen, Meeren etc.) möglicherweise sogar die vorherrschende Lebensform von Mikroorganismen. Sie verursachen auch in der Schifffahrt einen erheblichen wirtschaftlichen Schaden durch Bildung von Biofilmen auf den im Wasser befindlichen Teilen von Schiffen. Diese sind dann die organische Matrix für den Aufwuchs von Muscheln oder Bryozoen. Dieser Sekundäraufwuchs auf Schiffsrümpfen kann mehrere Dezimeter dick werden und zu einer drastischen Erhöhung des Wasserwiderstandes und damit zu einer Reduktion der Beweglichkeit und der Fahrtgeschwindigkeit der Schiffe und zu einem erhöhten Treibstoffverbrauch führen. Biofilme können aber auch für den Menschen bedrohlich werden. So stellen Biofilm-bildende humanpathogene Bakterien eine bedeutende Ursache für chronische und rekurrierende Infektionen in der Humanmedizin dar. Bekannte Beispiele hierfür sind die Plaquebildung auf Zähnen durch Streptokokken, die Alginatbildung von *Pseudomonas aeruginosa* bei Lungeninfeldionen im Rahmen einer cystischen Fibrose und nicht zuletzt die Besiedlung von Kunststoff- und Metallimplantaten durch Biofilm-bildende Staphylokokken in der modernen Intensivmedizin.

Im Hinblick auf die wachsende Bedeutung von nosokomialen Infektionen haben wir unsere Bestrebungen auf die Identifizierung von Biofilm-inhibierenden Wirkstoffen gegen multiresistente *Staphylococcus-aureus-* und *S.-epidermidis*-Erreger konzentriert. Diese Bakterien treten hauptsächlich im Zusammenhang mit der Verwendung von Kunststoff- und Metallimplantaten auf. Vor allem bei immunsupprimierten Patienten können sie schwere Allgemeininfektionen auslösen, die hauptsächlich von *Staphylococcus epidermidis* und *Staphylococcus aureus* verursacht werden. Beide Spezies bilden auf künstlichen Oberflächen (z.B. auf Venenkathetern, Herzschrittmachern oder auf Gelenkersatz) Biofilme aus, die aus den Bakterien selbst und einer Polysaccharidmatrix bestehen. Diese Matrix, die auch als **P**olysaccharid-**I**nterzelluläres-**A**dhäsin (PIA) bezeichnet wird, besteht aus □-1,6-verknüpften Glucosaminoglycan-Untereinheiten, die mit unterschiedlichen Seitengruppen substituiert sind (D. Mack, W. Fischer, A. Krokotsch, K. Leopold, R. Hartmann, H. Egge, R. Laufs, J. Bacteriol. 1996, 178, 175-183). Die Substanz vermittelt die Adhärenz der Zellen untereinander und ist damit für das dreidimensionale, mehrschichtige Wachstum eines Staphylokokken-Biofilms verantwortlich. Bisher konnte man vier Proteine, IcaA, IcaD, IcaB und IcaC, identifizieren, die in die PIA-Synthese involviert sind (C. Heilmann, O. Schweitzer, C. Gerke, N. Vanittanakom, D. Mack, F. Götz, Mol. Microbiol. 1996, 20, 1083-1091; C. Gerke, A. Kraft, R. Süssmuth, O. Schweitzer, F. Götz, J. Biol. Chem. 1998, 29, 18586-18593). Die Gene, die für diese Enzyme kodieren, sind im so genannten *icaADBC*-Operon organisiert, das bisher in allen getesteten *S.-aureus*-Isolaten und in 70 bis 80 Prozent aller *S.-epidermidis-*Stämme aus Fremdkörper-assoziierten Infektionen nachgewiesen wurde (W. Ziebuhr, C. Heilmann, F. Götz, P. Meyer, K. Wilms, E. Straube, J. Hacker, Infect. Immun. 1997, 65, 890-896; S. E. Cramton, C. Gerke, N. F. Schnell, W. W. Nichols, F. Götz, Infect. Immun. 1999, 67,5427-5433).

Obwohl das PIA nach allen bisherigen Erkenntnissen der wichtigste Faktor für den Aufbau eines Biofilms bei Staphylokokken ist, sind daran aber auch noch weitere Komponenten beteiligt. So wurde nachgewiesen, dass die Etablierung eines Biofilms in zwei Phasen abläuft. Die erste Phase erfordert zunächst die Adhärenz der Staphylokokken auf der Oberfläche, an die sich in der zweiten Phase die PIA-vermittelte Akkumulation des Biofilms anschließt. Die erste Phase der Biofilmbildung, die auch als initiale Adhärenz bezeichnet wird, wird bei *S*. *epidermidis* durch ein Oberflächenprotein vermittelt, das als AtlE bekannt ist (C. Heilmann, M. Hussain, G. Peters, F. Götz, Mol. Microbiol. 1997, 24, 1013-1024). AtlE übt neben der initialen Adhärenz noch eine weitere Funktion in der Staphylokokkenzelle aus. Es ist als Autolysin-Protein an der Separation der Zellwand bei der Zellteilung beteiligt. Mutationen im *atlE*Gen führten daher zur Hemmung der Biofilmbildung auf Oberflächen und zur Entstehung von Zellaggregaten im Kulturüberstand (C. Heilmann, M. Hussain, G. Peters, F. Götz, Mol. Microbiol. 1997, 24, 1013-1024). In jüngster Zeit wurden weitere Faktoren nachgewiesen, die an der Biofilmbildung von Staphylokokken beteiligt sind. Dazu gehören Teichonsäuren, die einen erheblichen Teil der Biofilm-Matrix ausmachen (I. Sadovskaya, E. Vinogradov, S. Flahaut, G. Kogan, S. Jabbouri, Infect Immun 2005, 73, 3007-3017.) Ebenso wurden zwei Oberflächen-assoziierte Proteine, Aap und Bap, identifiziert, die die akkumulativen Phase der Biofilmbildung, unabhängig von *ica* und PIA, vermitteln können (H. Rohde, C. Burdelski, K. Bartscht et al., Mol. Microbiol. 2005, 55, 1883-1895; C. Cucarella, C. Solano, J. Valle, B. Amorena, I. Lasa, J.R. Penades, J. Bacteriol. 2001, 183, 2888-2896.)

Ferner zeigte sich bei einigen der Verbindungen eine herausragende Aktivität gegen Trypanosomen. Diese einzelligen Parasiten sind wichtige Erreger in der Veterinär-, besonders aber in der Humanmedizin. Alljährlich leiden nach Angaben der Weltgesundheitsorganisation 300.000 bis 500.000 Menschen an der Schlafkrankheit, die von *Trypanosoma brucei* hervorgerufen wird (A. Stich, P.M. Abel, S. Krishna, BMJ. 2002, 325, 203-206). Die Krankheit endet ohne Therapie tödlich. Die derzeit verwendeten Medikamente sind nebenwirkungsreich, vielerorts nicht verfügbar und oftmals ungenügend wirksam. Neue medikamentöse Optionen werden deshalb dringend benötigt (A. Stich, M.P. Barrett, S. Krishna Trends Parasit. 2003, 19, 195-197).

Zhang Y et al. 2006 zeigen verschiedenen Synthesewege zur Herstellung von Isoquinolinen und ähnlichen Substanzen mittels einer Supersäure-gestützten Reaktion von N-Acyliminium Ionen.

Szantay C et al. 1965 untersuchen die tautomeren Formen der Basen die aus verschiedenen Isoquinolinium-Salzen freigesetzt werden können.

Beke D et al 1962 beschäftigen sich mit einer damals neuen Reaktion die zu einem Isochinolin-Ringschluss führt. Es wurde festgestellt, daß sich gewisse Tetrahydroisochinolinderivate nur aus C0 Methoxyl substituierte 2-Vinyl-benzaldehyden bilden.

Bringman G et al 2005 zeigen erstmals die vollständige Synthese des N,C gekoppelten Naphtylisoquinolinalkaloid Ancisheynin durch Kondensation eines monozyklischen Diketons oder des entsprechenden Benzopyrylium Salzes mit einem Aminonaphtalen.

Yang LK et al 2003, beschreiben die Gewinnung von Ancisheynin aus einem oberirdischen Teil von *Ancistrocladus heyneanus,* sowie seine strukturelle Analyse.

Neue Oxazolidinon-Derivate, die zur Bekämpfung von resistenten Schadorganismen verwendet werden sollen, werden in der WO 03/106454 beschrieben.

Es ist somit eine Aufgabe der vorliegenden Erfindung, neuartige, hoch wirksame und nicht toxische Substanzen zur Verfügung zu stellen, die insbesondere zu einer verbesserten Verhinderung der Biofilmbildung sowie zur Behandlung von Erkrankungen, wie z.B. Infektionskrankheiten verwendet werden können.

Diese Aufgabe wird durch die antiinfektive sowie Biofilm-hemmende Verbindungen der allgemeinen Formeln 1 bis 2 gelöst 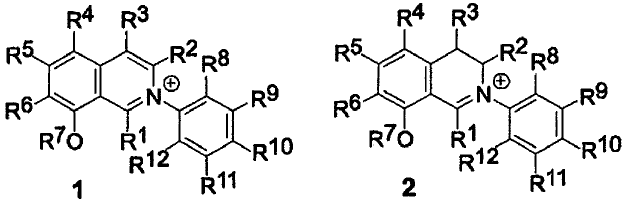 worin R³ H ist, R¹, R², R⁴ bis R⁶ und R⁸ bis R¹² unabhängig voneinander entweder H, ein unsubstituiertes, monosubstituiertes oder polysubstituiertes C₁-C₁₈-Alkyl, wobei das Alkyl gerade, verzweigt oder cyclisch sein kann, Alkenyl, ein unsubstituierter, monosubstituierter oder polysubstituierter Aryl- oder Heteroarylrest, eine unsubstituierte, monosubstituierte oder polysubstituierte Benzylgruppe, eine Acylgruppe, wie z.B. Formyl, Acetyl, Trichloracetyl, Fumaryl, Maleyl, Succinyl, Benzoyl, oder eine verzweigte oder Heteroatom- oder arylsubstituierte Acylgruppe, ein Alkoxysubstituent, wie z.B. -OMe, -OEt, -O*n*Pr, -*i*Pr, -O*n*Bu, -O*i*Bu,-*OsecBu,* -O*t*Bu, dessen Alkylgruppe verzweigt, unverzweigt oder cyclisch ist, eine über ein Schwefelatom gebundene Alkylgruppe, wie z.B. -SMe, -SEt, oder eine Sulfonylgruppe, wie z. B. -SO₃H, -SO₂Me, -SO₂CF₃, -SO₂C₆H₄CH₃ oder SO₂C₆H₄CH₂Br, oder ein Stickstoff-substituent, wie z.B. NH₂, NHR, NRR' (mit R, R' = Alkyl, Aryl usw.), -NC oder NO₂, oder Fluor, Chlor, Brom, Jod, -CN ist,

R⁷ unabhängig voneinander entweder H, ein unsubstituiertes, monosubstituiertes oder polysubstituiertes C₁-C₁₈-Alkyl, wobei das Alkyl gerade, verzweigt oder cyclisch sein kann, ein monosubstituiertes oder polysubstituiertes, gerades, verzweigtes oder cyclisches C₁-C₁₈-Alkenyl oder eine Acylgruppe, wie z.B. Formyl, Acetyl, Trichloracetyl, Fumaryl, Maleyl, Succinyl, Benzoyl, verzweigte oder Heteroatom- oder arylsubstituierte Acylgruppen sein kann,

und R⁸ bis R¹² auch in der Art verknüpft sein können, dass dadurch ein unsubstituierter, monosubstituierter oder polysubstituierter Ring und Dimere von **1** entsteht, sowie pharmazeutisch verträgliche Salze oder Solvate dieser Verbindung, mit der Maßgabe, dass Formel 1-2 nicht die folgenden Formeln 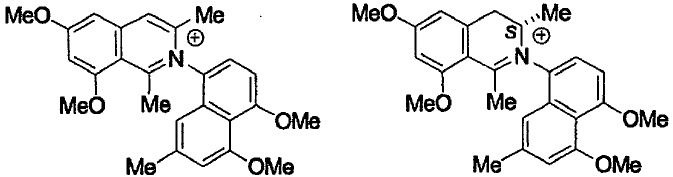 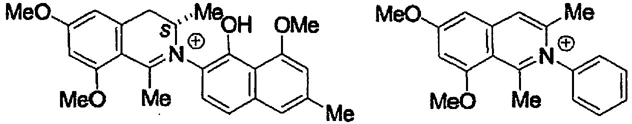 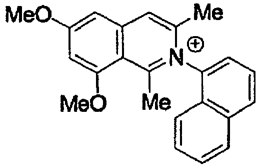 bedeuten, zur Behandlung und/oder Prävention von Erkrankungen.

Im Hinblick auf die wachsende Bedeutung von Krankenhausinfektionen haben die Erfinder ihre Bestrebungen auf die Identifizierung von neuen Wirkstoffen gegen multiresistente *Staphylococcus-aureus-* und S *-epidermidis-Erreger* konzentriert, da diese für die größte Zahl an Krankenhausinfektionen verantwortlich sind. Es wird dabei nicht nur der Ansatz der bakteriostatischen oder bakteriziden Abtötung der Erreger wirksame verfolgt, sondern es werden vor allem auch solche Stoffe gesucht, die in die Genregulation und Genexpression von Virulenzfaktoren eingreifen. Dieses Konzept erscheint insbesondere bei Frerndkörper-assoziierten Staphylokokken-Infektionen sinnvoll. Staphylokokken bilden auf Kunststoff- und Metalloberflächen medizinischer Implantate Biofilme aus, die eine wichtige Quelle für persistierende und rekurrierende Infektionen darstellen. Eine Verhinderung der Biofilmbildung oder deren Auflösung und Beseitigung würde erheblich zur Bekämpfung nosokomialer Infektionen beitragen.

Ein weiterer Aspekt der vorliegenden Erfindung betrifft die Verwendung einer Reihe der oben genannten Verbindungen zur Behandlung von Infektionskrankheiten, wie Leishmaniose und Trypanosomen-Erkrankungen (wie die afrikanische Schlafkrankheit oder der Chagas-Krankheit). So zeigt sich, daß sich bei Veränderung der verschiedenen strukturellen Parameter günstiger weise die Selektivität der Aktivität spezifisch gegen nur einen bestimmten Erreger verbessern läßt.

Eine medizinische Verwendbarkeit der erfindungsgemäß aufgefundenen Verbindungen der allgemeinen Formeln **1** bis **2** war bisher unbekannt. Ein weiterer Aspekt der vorliegenden Erfindung betrifft somit deren Verwendung zur Prävention oder Behandlung von Erkrankungen, wie z.B. Tumorkrankheiten oder Infektionskrankheiten.

Ein weiterer Aspekt der vorliegenden Erfindung betrifft dann die Verwendung einer Verbindung der allgemeinen Formeln 1 bis **2:** 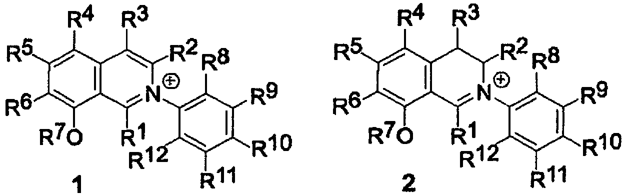 wobei die Reste R¹ bis R¹² wie oben definiert sind, zur Verhinderung der Biofilmbildung auf Oberflächen. Bevorzugterweise betrifft die Verwendung eine Verhinderung der Biofilmbildung durch Staphylokokken, wie etwa *S. epidermidis,* auf Kunststoff- und Metalloberflächen medizinischer Implantate, Stents, Katheter, Kanülen und anderer medizinischer invasiver Vorrichtungen.

Ein Aspekt der Beschreibung betrifft die Verwendung der erfindungsgemäßen Verbindungen als Werkzeuge zur Untersuchung und Erforschung der Biofilmbildung und als "Lead Structures" (Leitstrukturen) für die Entwicklung weiterer Biofilmbildung-verhindernder sowie antiinfektiver Substanzen.

Im Rahmen der vorliegenden Beschreibung soll unter einem "Derivat" eine von der allgemeinen Formeln 1 bis 2 abgeleitete Verbindung sein, die zum Beispiel durch verschiedene der oben für R₁ bis R₁₂ angegebenen Restgruppen substituiert ist, sowie Gemische verschiedener dieser Verbindungen, die zum Beispiel zu einem auf die jeweils zu therapierende Erkrankung und/oder den Patienten auf Basis von diagnostischen oder Daten über den Behandlungserfolg oder -verlauf abgestimmten "personalisierten" Medikament verarbeitet werden können.

Unter einem "Vorläufer" einer Substanz soll im Rahmen der vorliegenden Erfindung zum einen eine Substanz verstanden werden, die im Laufe ihrer Verabreichung zur Behandlung durch die Bedingungen im Körper (z.B. pH im Magen, oder ähnliches) so verändert wird, oder aber durch den Körper nach Aufnahme so metabolisiert wird, dass sich als wirksame Substanz die erfindungsgemäße Verbindung oder deren Derivate bilden.

Die Erfindung soll nun im Folgenden weiter in den Beispielen unter Bezug auf die beigefügte Figur beschrieben werden, ohne jedoch auf diese begrenzt zu sein. Gegenstände die nicht von den Ansprüchen erfaßt sind dienen lediglich der Illustration.

### Beispiel 1:

### Synthese des Isochinolinium-Salzes A (= Formel 1a)

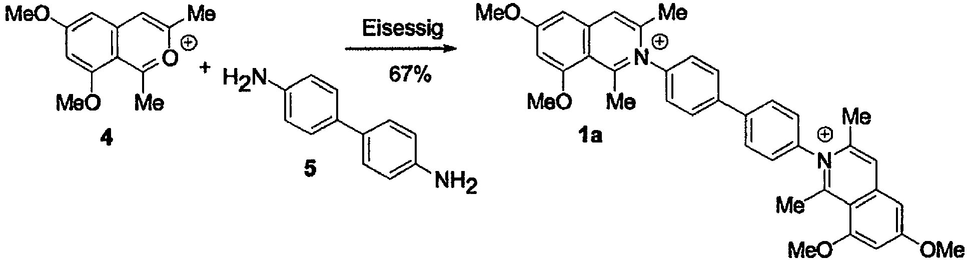

200 mg (0.628 mmol) des Benzopyrylium-Salzes **4** (hergestellt nach G. Bringmann, *Liebigs Ann. Chem.* **1985,** 2126-2134) wurden in 5 ml Eisessig gelöst, mit 57.9 mg (0.314 mmol) Benzidin (5) versetzt und über Nacht bei Raumtemperatur gerührt. Nach beendeter Reaktion wurden zu der Suspension 5 ml Diethylether gegeben und der ausgefallene Feststoff abgesaugt. Das Lösungsmittel der Mutterlauge wurde im Vakuum entfernt und der ölige Rückstand an Sephadex-LH20 säulenchromatographisch gereinigt (Methanol + 5% Trifluoressigsäure). Man erhielt das Isochinolinium-Salz A in Form beiger Nadeln.

### N,N'-(1,1'-Benzidin)-di-(6,8-dimethoxy-1,3-dimethylisochinolinium)-Salz (A)

Ausbeute: 171 mg (0.210 mmol; 67%).

Schmelzpunkt: > 350 °C (Methanol)

IR (KBr): *ṽ*= 3409 (br), 2949 (m), 2823 (w), 1687 (m), 1643 (m), 1612 (s), 1559 (m), 1494 (w), 1466 (m), 1387 (s), 1288 (w), 1201 (s), 1116 (s), 1027 (m), 970 (w), 837 (w), 799 (w) cm⁻¹

¹H-NMR (400 MHz, DMSO): δ = 2.29 (s, 6H, 3-CH₃), 2.91 (s, 6H, 1-CH₃), 4.17 (s, 12H, OCH₃), 7.09 (d, ⁴*J* = 2.15 Hz, 2H, Ar-H), 7.21 (d, ⁴*J =* 2.15 Hz, 2H, Ar-H), 7.78 (d, ³*J* = 8.59 Hz, 4H, Ar-H), 8.15 (s, 2H, Ar-H), 8.28 (d, *³J* = 8.59 Hz, 4H, Ar-H) ppm.

¹³C-NMR (100 MHz, DMSO): δ = 21.63 (CH₃), 23.27 (CH₃), 56.62 (OCH₃), 57.12 (OCH₃) 98.93, 102.2, 110.19, 121.68, 127.45, 129.10, 139.41, 140.21, 141.78, 144.05, 59.45, 161.36, 166.79 (Ar-C) ppm.

MS (70 eV): *m*/*z* (%) 586 (14) [M]⁺, 369 (10), 353 (8), 293 (24), 264 (8), 69 (13), 44 (49).

C₃₈H₃₈N₂O₄ (HRMS): Ber. 586.2823 Gef. 586.2785

### Beispiel 2:

### Synthese des Dihydroisochinolinium-Salzes 2a

### Darstellung des sekundären Amins (S)-8

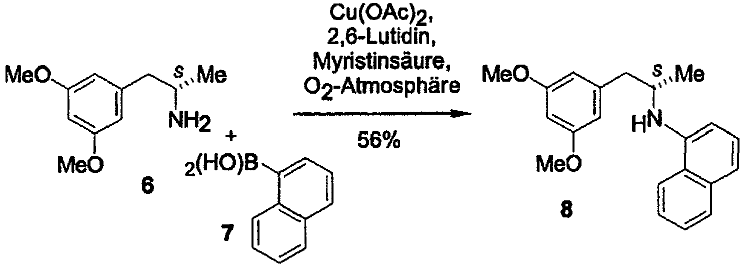

272 mg (2.55 mmol) 2,6-Lutidin wurde zu einer Suspension bestehend aus 658 mg (3.83 mmol) 1-Naphthylboronsäure (7), 0.926 mg (0.005 mmol) Kupfer(II)acetat und 1.14 mg Myristinsäure in 5 ml absolutierten Toluol gegeben. Nach 5 Minuten wurden 500 mg (2.55 mmol) primäres Amin 6 (hergestellt nach G. Bringmann, R. Weirich, H. Reuscher, J. R. Jansen, L. Kinzinger, T. Ortmann Liebigs Ann. Chem. 1993, 877-888) zugegeben und die Reaktionsmischung 30 Stunden lang unter einer Sauerstoffatmosphäre bei Raumtemperatur gerührt. Anschließend wurde die Suspension mit 10 ml Essigsäureethylester verdünnt und über Kieselgel (Essigsäureethylester) filtriert. Das Lösungsmittel wurde im Vakuum entfernt und das Produkt mittels Säulenchromatographie (Kieselgel, Petrolether:Ethylacetat = 10:1) gereinigt. Es wurde das sekundäre Amin 8 als braunes Öl erhalten.

### (2R)-N-(1-Naphthyl)-1-(3',5'-dimethoxyphenyl)-2-aminopropan (S)-8

Ausbeute: 459 mg (1.43 mmol; 56%).

Drehwert: α_{D} = 47 ° (c = 0.10, Dichlormethan)

IR (KBr): *ṽ* = 3417 (br), 3098 (m), 2997 (m), 2918 (w), 1595 (s), 1523 (w), 1459 (m), 1406 (w), 1385 (w), 1276 (m), 1203 (m), 1149 (s), 1082 (w), 792 (w), 769 (s) cm⁻¹.

¹H-NMR (400 MHz, MeOD): δ = 1.32 (d, ³*J* = 6.44 Hz, 3H, CH₃), 2.85 (dd, ³*J* = 13.26 Hz, ²*J* = 7.08 Hz, 1 H, CH₂), 3.00 (dd, ³*J* = 13.27 Hz, ²*J* = 5.30 Hz, 1H, CH₂), 3.69 (s, 6H, OCH₃), 3.98 (m, 1H, CH), 6.31 (t, 1H, Ar-H), 6.43 (d, ⁴*J* = 2.27 Hz, 2H, Ar-H), 6.74 (d, ³*J* = 7.58 Hz, 1H Ar-H), 7.18 (d, ³*J* = 8.21 Hz, 1H, Ar-H), 7.34 (t, 1H, Ar-H), 7.42 (m, 2H, Ar-H), 7.77 (dd, ³*J* = 9.47 Hz, ⁴*J* = 2.02 Hz, 1H, Ar-H), 7.96 (dd, ³*J* = 6.57 Hz, ⁴*J* = 1.64 Hz, 1H, Ar-H) ppm.

¹³C-NMR (100 MHz, MeOD): δ = 20.48 (CH₃), 43.10 (CH₂), 50.87 (CH), 55.73 (OCH₃), 99.50, 106.1, 108.7, 117.8, 121.9, 125.3, 125.4, 126.7, 127.8 129.5, 136.3, 142.8, 144.3, 162.4 (Ar-C) ppm.

MS (70 eV): *m*/*z* (%) 322 (2) [M+H]⁺, 221 (10) [M]⁺, 234 (2), 170 (100), 154 (6), 128 (7), 115 (3), 91 (2), 77 (3), 42 (2).

C₂₁H₂₄NO₂ (HRMS): Ber. 322.18070 Gef. 322.18043

### Darstellung des Amids (S)-9

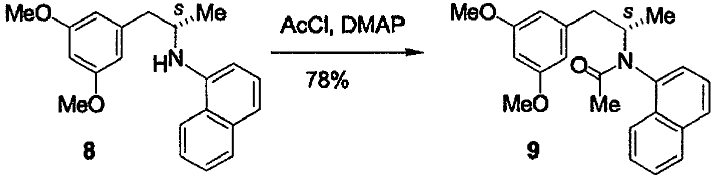

Bei Raumtemperatur wurden unter Schutzgasatmosphäre zu einer Lösung bestehend aus 256 mg (0.731 mmol) sekundärem Amins **8** und 120 mg (0.877 mmol) DMAP in 15 ml Toluol 138 mg (1.76 mmol) Acetylchlorid zugetropft und 12 Stunden lang unter Rückfluss erhitzt. Die abgekühlte Reaktionsmischung wurde mit Wasser versetzt und mit Essigsäureethylester extrahiert. Die vereinigten organischen Phasen wurden mit MgSO₄ getrocknet und das Lösungsmittel im Vakuum entfernt. Der Rückstand wurde an Kieselgel mit Petrolether:Essigsäureethylester (3:1) chromatographiert. Man erhielt das Amid 9 in Form seiner beiden Konformere (1:0.7) in Form beiger Plättchen.

### (2R)-N,N-(1-Naphthyl-acetyl)-1-(3',5'-dimethoxyphenyl)-2-aminopropan (S)-9

Ausbeute: 223 mg (0.615 mmol; 84%)

Schmelzpkt: 115 °C (Petrolether:Essigsäureethylester)

Drehwert: α_{D} = 18° (c = 0.10, Methanol)

IR (KBr): *ṽ* = 3428 (br), 2961 (s), 2838 (w), 1657 (s), 1593 (s), 1507 (w), 1463 (s), 1428 (m), 1399 (m), 1380 (m), 1341 (w), 1320 (w), 1282 (s), 1240 (m), 1204 (m), 1159 (s), 1054 (m), 1016 (m), 926 (w), 837 (m), 805 (m), 782 (s), 684 (m), 601 (s) cm⁻¹.

¹H-NMR (400 MHz, CDCl₃): δ = 0.78 (d, ³*J* = 6.94 Hz, 3H, CH₃), 0.95 (d, ³*J* = 6.94 Hz, 2H, CH₃), 1.70 (s, 5H, CH₃CO), 2.25 (dd, ³*J* = 13.01Hz, ²*J* = 9.98 Hz, 0.7 H, CH₂), 2.54 (dd, ³*J* = 12.63 Hz, ²*J* = 9.98 Hz, 1H, CH₂), 3.19 (dd, ³*J* = 13.01, ²*J* = 5.43 Hz, 0.7 H, CH₂), 3.31 (dd, ³*J* = 12.75 Hz, ⁴*J* = 4.29 Hz, 1H, CH₂),3.72 (s, 4.2 Hz, OCH₃), 3.81 (s, 6H, OCH₃), 4.89 (m, 0.7H, CH), 5.09 (m, 1H, CH), 6.27 (d, ⁴*J* = 2.02 Hz, 1.4H, Ar-H), 6.26 (t, 0.7H, Ar-H), 6.32 (t, 1H, Ar-H), 6.49 (d, ⁴*J* = 2.28, 1H, Ar-H), 7.13 (d, ³*J* = 6.19 Hz, 0.7H, Ar-H), 7.29 (d, ⁴*J* = 1.01Hz, 1H, Ar-H),7.42 - 7.60 (m, 5H, Ar-H), 7.81 - 7.99 (m, 5H, Ar-H) ppm.

¹³C-NMR (100 MHz, CDCl₃): δ = 16.79 (CH₃), 19.24 (CH₃), 23.18 (CH₃), 23.33 (CH₃), 41.78 (CH₂), 43.54 (CH₂), 55.63 (OCH₃), 55.70 (OCH₃), 55.72 (CH), 57.55 (CH), 99.56, 99.63, 108.2, 108.3, 124.11, 124.12, 126.71, 126.74, 127.7, 127.8, 128.31, 128.37, 128.41, 128.5, 129.7, 129.8, 30.3, 130.4, 132.9, 133.3, 136.18, 136.20, 137.6, 138.6, 142.56, 142.63 ppm.

MS (70 eV): *m*/*z* (%) 363 [M]⁺ (2), 185 (76), 170 (100), 156 (32), 143 (38), 127 (23), 115 (15),49 (17), 43 (38).

C₂₃H₂₅NNaO₃ (HRMS): Ber. 386.17266 Gef. 386.17270

### Darstellung des Dihydroisochinolinium-Salzes (S)-2a

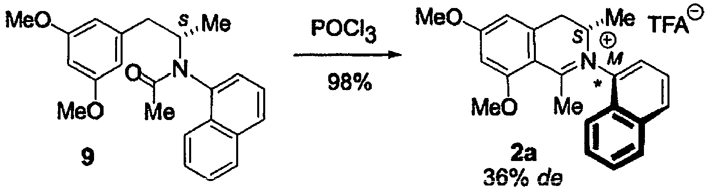

7.00 mg Amid 9 in 2 ml absolutiertem Acetonitril wurden bei Raumtemperatur mit 0.01 ml POCl₃ versetzt und 1 Stunde lang zum Refluxieren erhitzt. Nach Abkühlen der Reaktionsmischung auf Raumtemperatur wurde der Reaktionsmischung Wasser+TFA (1%) zugesetzt und das Acetonitril im Vakuum entfernt. Die verbleibende wässrige Suspension wurde mit Essigsäureethylester extrahiert, die organischen Phasen vereinigt, mit MgSO₄ getrocknet und das Lösungsmittel entfernt. Der Rückstand wurde mittels Gelchromatographie (Sephadex, Methanol) gereinigt, wodurch man das Isochinolinium-Salz (*M*)-**2a** und sein Atropdiastereomer (P)-**2a** (1:0.4) als gelbes Öl erhielt. Die beiden Diastereomere konnten mittels HPLC getrennt werden (Waters Symmetry C18, 45% Wasser + 0.05% TFA, 55% Methanol + 0.05% TFA, isokratisch, 0.7 ml/min: t*_{R}* = 8.1 min (*M-***2a**), t*_{R}* = 8.7 min (*P*-**2a**)).

### (3S)-N,1'-Naphthyl-6,8-dimethoxy-1,3-dimethyl-3,4-dihydroisochinolinium-Trifluoro-acetat (M)-2a und (P)-2a

Ausbeute: 8.78 mg (0.019 mmol; 98%)

Drehwert: α_{D} = 6° (c = 0.25, Methanol)

IR (KBr): *ṽ* = 3437 (br), 3020 (m), 2934 (m), 2843 (w), 1650 (s), 1595 (s), 1508 (w), 1462 (m), 1428 (m), 1379 (m), 1323 (m), 1288 (m), 1204 (m), 1152 (s), 1057 (m), 928 (w), 833 (w), 807 (m), 780 (s), 686 (m) cm⁻¹.

¹H-NMR (400 MHz, MeOD): δ = 1.29 (d, ³*J* = 6.82 Hz, 1.3H, 3-CH₃), 1.40 (d, ³*J =* 6.83 Hz, 3H, 3-CH₃), 2.52 (s, 1.3H, 1-CH₃), 2.58 (s, 3H, 1-CH₃), 3.21 (dd, *J =* 16.8 Hz, *J =* 2.53 Hz, 1H, CH₂), 3.27 (dd, *J =* 20.97 Hz, *J =* 4.67 Hz, 0.4H, CH₂), 3.80 (dd, *J =* 15.8 Hz, *J =* 5.81Hz, 0.4H, CH₂), 3.96 (dd, *J =* 16.8 Hz, *J =* 6.19 Hz, 1H, CH₂), 3.98, 4.81 (s, 8.4H, OCH₃), 4.39 (m, 1H, CH), 4.66 (m, 0.4H, CH), 6.81 (m, 1.4H, Ar-H), 6.86 (m, 1.4H, Ar-H), 7.69 - 7.88 (m, 6H, Ar-H), 7.98 (d, *J =* 8.33 Hz, 1H, Ar-H), 8.18 (m, 1.4H, Ar-H), 8.26 (d, *J =* 8.08 Hz, 1.4Hz, Ar-H) ppm.

¹³C-NMR (100 MHz, MeOD): δ = 16.79 (OCH₃), 19.24 (CH₃), 23.18 (CH₃), 23.33 (CH₃), 41.78 (CH₂), 43.54 (CH₂), 55.63 (OCH₃), 55.70 (OCH₃), 55.72 (CH), 57.55 (CH), 99.56, 99.63, 108.2, 108.3, 124.11, 124.12, 126.71, 126.74, 127.7, 127.8, 128.31, 128.37, 128.41,

128.5, 129.7, 129.8, 130.1, 130.3, 130.5, 130.7, 132.3, 132.5, 134.0, 135.8, 136.5, 136.20, 138.1, 142.56, 142.1, 142.70, 142.72, 166.6, 170.8, 170.7, ppm.

MS (70 eV): *m*/*z* (%) 369 [M+Na]⁺ (2), 353 (M+Na-CH₄), (3), 212 (22), 185 (52), 170 (100), 154 (7), 143 (16), 127 (5), 43 (7).

MS (ESI): 346.6

### Beispiel 3:

### Synthese des Tetrahydroisochinolins 3a

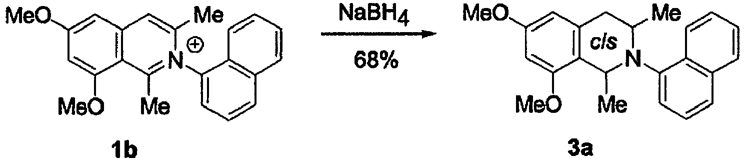

Zu einer Lösung von 56.0 mg (0.126 mmol) des Isochinolinium-Perchlorates **1b** in 14 ml Methanol wurden 57.2 mg (0.151 mmol) NaBH₄ portionsweise bei 0 °C zugegeben. Nach einstündigem Rühren der Reaktionsmischung bei Raumtemperatur wurden 5 ml Wasser hinzugefügt und weitere 12 Stunden gerührt. Die Suspension wurde anschließend mit halbkonzentrierter Salzsäure versetzt und das Produkt mit Diethylether extrahiert. Die etherischen Phasen wurden vereinigt, mit MgSO₄ getrocknet und das Lösungsmittel im Vakuum entfernt. Der ölige Rückstand wurde an Kieselgel säulenchromatographisch (Hexan:Ethylacetat 1:2) gereinigt. Man erhielt das Naphthyltetrahydroisochinolin **3a** als weißen Feststoff.

### N-(1-Naphthyl)-6,8-dimethoxy-1,3-(cis)-dimethyltetrahydroisochinolin 3a

Ausbeute: 30.0 mg (0.103 mmol; 68%).

Schmelzpunkt: 69°C (Hexan:Ethylacetat)

IR (KBr): *ṽ* = 3432 (br), 3043 (s), 2990 (s), 2926 (s), 2835 (m), 1687 (m), 1608 (s), 1490 (w), 1459 (m), 1423 (w), 1391 (w), 1364 (w) 1341 (w), 1321 (w), 1295 (w), 1260 (m), 1234 (w), 1207 (m), 1150 (s), 1109 (s), 1094 (s), 1049 (s), 1023 (s), 941 (w), 826 (w), 802 (m), 779 (s) cm⁻¹.

¹H-NMR (400 MHz, CDCl₃): δ = 0.87 (d, ³*J* = 6.31 Hz, 3H, 3-CH₃), 2.91 (d, ³*J* = 6.32 Hz, 3H, 1-CH₃), 2.77 (dd, ³*J* = 15.29 Hz, ²*J* = 2.90 Hz, 1H, CH₂), 2.89 (dd, ³*J* = 15.03 Hz, ²*J* = 9.35 Hz, 1H, CH₂), 3.42 (m, 1H, 3-CH), 3.78 (s, 3H, OCH₃), 3.86 (s, 3H, OCH₃), 4.57 (q, 1H, 1-CH), 6.32 (d, ⁴*J* = 2.28 Hz, 1H, Ar-H), 6.58 (d, ⁴*J =* 2.28 Hz, 1H, Ar-H), 7.40 - 7.48 (m,

5H, Ar-H), 7.68 (d, ³*J* = 7.71 Hz, 1H, Ar-H), 7.82 (dd, ³*J* = 6.06 Hz, ⁴*J* = 3.41 Hz, 1H, Ar-H) ppm.

¹³C-NMR (100 MHz, CDCl₃): δ = 24.11 (CH₃), 28.37 (CH₃), 39.88 (CH₂), 55.45 (CH), 55.89 (OCH₃), 56.07 (OCH₃), 97.34, 104.7, 109.2, 122.8, 124.8, 125.3, 125.6, 126.0, 126.3, 126.5, 126.9, 128.5, 128.7, 138.5, 157.9, 159.4 (Ar-C).

MS (70 eV): *m*/*z* (%) 347 (5) [M]⁺, 332 (100) [M-CH₃]⁺, 316 (7) [M-OCH₃]⁺, 189 (8) [M-Naphthyl]⁺, 127 (17) [Naphthyl]⁺, 40 (12).

| | | | | |
|---|---|---|---|---|
| C₂₃H₂₅NO₂ (347.4501) | Ber. | C 79.51 | H 7.25 | N 4.03 |
| | Gef. | C 79.29 | H 7.59 | N 3.87 |

### Beispiel 4:

### Biologische Aktivitäten

### 1. Wirkung gegen Parasiten der Gattung Leishmania

*Leishmania major*-Promastigoten (MHOM/IL81/FE/BNI) wurden in Blutagar-Kulturen bei 26°C, 5% CO₂ und 95% Luftfeuchtigkeit kultiviert. Für das Experiment wurden die Promastigoten zweimal mit salinem Phosphatpuffer (PBS) gewaschen und anschließend (10⁸ Zellen/ml) in Click-RPMI-1640-Medium ohne Phenolrot, versetzt mit 10% FCS, 2 mM L-Glutamin, 10 mM HEPES-Puffer, pH 7.2, 100 µg/ml Penicillin, 160 mg/ml Gentamicin, 7.5% NaHCO₃ und 50 µM 2-Mercaptoethanol (Kulturmedium) suspensiert. Zur Aktivitätsbestimmung wurden jeweils 200 µl der Promastigoten-Suspension in Mikrotiterplatten mit den Substanzen in einer geometrischen Verdünnungsreihe 24 Stunden lang, bei 26°C, 5% CO₂ und 95% Luftfeuchtigkeit inkubiert. Anschließend wurde jeder Mikrokultur 20 µl Alamar Blue (Trinova Biochem, Gießen, Deutschland) hinzugefügt und die Kulturen weiter inkubiert. Kulturen, die nur Medium und die Substanz oder nur die Zellsuspension (ohne Substanz) enthielten, dienten als Kontrollen. Die optische Dichte wurde nach weiteren 24 Stunden mit einem ELISA-Reader bei den Wellenlängen 550 nm und 630 nm gemessen. Die IC₅₀-Werte der Substanzen wurden durch lineare Interpolation (J. Mikus, D. Steverding. Parasitol. Int. 2000, 48, 265-269) berechnet.

Macrophagen der Zelllinie J774.1 wurden gewaschen und in Kulturmedium suspensiert (2 x 10⁸ Zellen/ml). Für den Test wurden 200 µl der Zellsuspension und die Substanzen in einer geometrischen Verdünnungsreihe in Kulturen von Mikrotiterplatten pipettiert. Nach 24 Stunden Inkubation bei 37°C, 5 % CO₂ und 95% Luftfeuchtigkeit wurde jeder Mikrokultur 20 µl Alamar Blue zugefügt und die Mikrotiterplatte weiter inkubiert. Mikrokulturen mit Medium und Substanz sowie Mikrokulturen, die nur Zellsuspension (ohne Substanz) enthielten, dienten als Kontrollen. Die optische Dichte wurde nach 24, 48 und 72 Stunden mit einem ELISA-Reader bei den Wellenlängen 550 nm und 630 nm gemessen. Der IC₅₀-Wert der Substanzen wurde durch lineare Interpolation berechnet. Amphotericin B diente als Referenzsubstanz und positive Kontrolle.

Die Aktivitäten der Verbindungen der allgemeinen Strukturen **1-3** gegen Leishmanien wurden an einer Reihe von Beispielen nach dem oben beschriebenen Verfahren untersucht. Der therapeutische Index entspricht dem Verhältnis von Zytoxizität gegen Wirtszellen (J774.1 Makrophagen) zu Aktivität gegen *Leishmania major.*

**Tabelle 1: Exemplarische Wirkung gegen Leishmania major sowie deren therapeutischer Index.**

| **Beispiele für Testsubstanzen** | **IC₅₀ (µM)** | **Therapeutischer Index** |
|---|---|---|
| 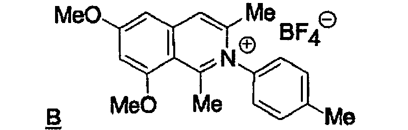 | 2.45 | 13 |
| 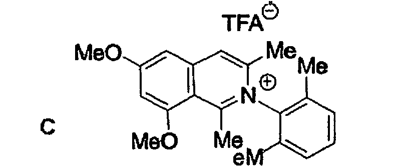 | 2.02 | 17 |
| 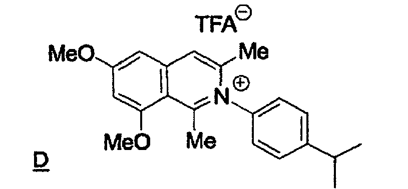 | 0.85 | 18 |
| Amphotericin B | 2.51 | n.d. |

Die in Tabelle 1 exemplarisch gezeigten Verbindungen zeigen eine ausgezeichnete Wirkung gegen *L. major.* Hierbei weisen gerade die Substanzen mit Alkyl-Substituenten im Aryl-Rest eine sehr gute, vor allem selektive Wirkung gegen diesen Erreger auf. Substanz D dient dabei als bevorzugte Leitsubstanz, die sich auch zu einer weiteren chemischen Derivatisierung eignet.

### 2. Anti-trypanosomiale Wirkungen

Es wurden Blutstromformen des TC-221-Stammes von *Trypanosoma brucei brucei* in angereichertem Baltz-Nährmedium in einer Atmosphäre von 5 % CO₂ bei 37 °C kultiviert. Zur Aktivitätsbestimmung wurde eine definierte Erregerzahl (10⁴ Trypanosomen pro ml) in 96-Lochplatten mit den Substanzen in unterschiedlicher Konzentration über 48 bis 72 Stunden inkubiert. Der Effekt der Wirkstoffe war über die ED₅₀-Werte durch lineare Interpolation quantifizierbar. Die trypanozide Aktivität der Testsubstanzen wurde über Absorptionsmessungen am MR 700 Microplate Reader (Testwellenlänge 550 nm, Referenzwellenlänge 630 nm) mittels Alamar Blue®, einem Indikator metabolischer Zellfunktionen, bestimmt. Die Zugabe des Farbstoffes erfolgte 24 Stunden nach Beginn der Inkubation. Der bei der Absorptionsmessung genutzte Farbumschlag von Alamar Blue® beruht auf Reduktionsprozessen, in welchen NADH oder NADH-abhängige Enzyme involviert sind. Inkubationszeiten und die benötigte Farbstoffkonzentration korrelieren mit der Stoffwechselaktivität der Trypanosomen. Der MIC-Wert *(minimum inhibitory concentration*) wurde mikroskopisch durch Auszählen lebender Zellen in Neubauer-Zählkammern ermittelt. Als Positiv-Kontrolle und Referenz dienten Suramin Na.

Die anti-trypanosomale Aktivität der Verbindungen der allgemeinen Strukturen **1-3** wurde an einer Reihe von Beispielen nach dem oben beschriebenen Verfahren untersucht. Der therapeutische Index entspricht dem Verhältnis der Cytoxizität (J774.1 Macrophagen) gegen die Aktivität gegen *Trypanosoma brucei brucei.*

**Tabelle 2: Exemplarische Wirkung gegen Trypanosoma brucei brucei nach 48 und 72 Stunden sowie deren therapeutischer Index.**

| **Beispiele für Testsubstanzen** | **ED₅₀ (µM) nach 48h** | **ED₅₀ (µM) nach 72h** | **Therapeutischer Index** |
|---|---|---|---|
| 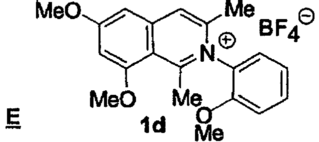 | 0.383 | 0.323 | 86 |
| 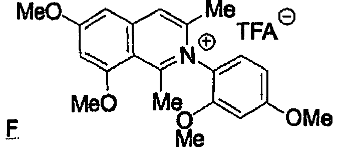 | 0.325 | 0.311 | 72 |
| 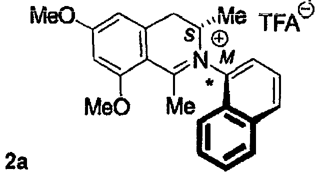 | 0.390 | 0.356 | > 180 |
| Suramin Na | 0.31 | 0.32 | n.d. |

Wie die in Tabelle 2 beispielhaft gezeigten Substanzen besitzen eine ausgeprägte Wirkung gegen *T*. *brucei brucei.* Im In-vitro-Modell wiesen gerade die Verbindungen mit elektronenschiebenden Substituenten im *N*-Aryl-Substituenten eine selektive Wirkung gegen diesen Erreger auf, bei guten therapeutischen Indices. Bei Testungen dieser Verbindungen gegen weitere Zellen, wie L5178Y Mäuselymphom-Zellen, zeigten sich ebenfalls vergleichsweise geringe Cytotoxizitäten (ED₅₀ > 10 µM), dann wenn die Zellen unter Wachstumsbedingungen gehalten werden, bei denen die Generationsdauer von 12 Stunden auf 18 Stunden erhöht wird. Substanz 2a dient dabei als bevorzugte Leitsubstanz, die sich auch zu einer weiteren chemischen Derivatisierung eignet.

### 3. Anti-plasmodiale Wirkungen

Für die Bestimmung der anti-plasmodialen Aktivitäten wurden Kulturen des *Plasmodium falciparum* Stammes K1 (resistent gegen Chloroquin und Pyrimethamin) verwendet. Es wurde eine Modifikation (R. G. Ridley, W. Hofheinz, H. Matile, C. Jacquet, A. Dorn, R. Masciadri, S. Jolidon, W. F. Richter, A. Guenzi, M. A. Girometta, H. Urwyler, W. Huber, S. Thaitong, W. Peters, Antimicrob. Agents Chemother. 1996, 40, 1846-1854) des [³H]-Hypoxanthin-Einbau-Tests (R. E. Desjardins, C. J. Canfield, D. Haynes, J. Chulay, Antimicrob. Agents Chemother. 1979, 16, 710-718) verwendet. Mit *P. falciparum* infizierte menschliche rote Blutzellen (0.3% Infektion) bei einem Haematokrit von 2.5% (Volumenanteil der roten Blutzellen) in RPMI 1640 Medium mit dem Serumersatz Albumax II (5g/L) wurden mit seriellen Verdünnungsreihen der Wirkstoffe in Mikrotiterplatten 48 Stunden lang bei 37 °C, unter einer CO₂-angereicherten und O₂-reduzierten Atmosphäre inkubiert. Dann wurde zu jedem Well der Mikrotiterplatte 0.5 µCi [³H]-Hypoxanthin hinzugegeben, und nach weiteren 24 Stunden Inkubationszeit wurden die Wells mit einem Betaplate Cell Harvester auf Glasfaserfiltern abgeerntet und mit destilliertem Wasser lysiert. Die Inkorporation von radiomarkiertem Hypoxanthin (die mit der Quantität lebender Parasiten korreliert) wurde mit Hilfe eines Szintillationszählers bestimmt. Der IC₅₀ Wert wurde aus sigmoidalen Inhibitionskurven bestimmt. Die Tests wurden im Duplikat durchgeführt und mindestens einmal wiederholt. Als Kontrolle (100% Inkorporation) dienten Parasitenkulturen ohne Zusatz, als Positivkontrolle Kulturen mit einer Verdünnungsreihe von Chloroquin.

Die anti-plasmodiale Aktivität der Verbindungen der allgemeinen Strukturen **1-3** wurde an einer Reihe von Beispielen nach dem oben beschriebenen Verfahren untersucht. Der therapeutische Index entspricht dem Verhältnis der IC₅₀ Werte für Rattenmyoblasten (L-6-Zellen = Cytotoxizität) zu den IC₅₀ Werten für *Plasmodium falciparum.*

**Tabelle 3: Exemplarische Wirkung gegen Plasmodium falciparum sowie deren therapeutischer Index**

| **Beispiele für Testsubstanzen** | **IC₅₀ (µM)** | **Therapeutischer Index** |
|---|---|---|
| 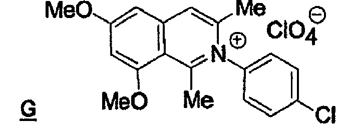 | 0.056 | 671 |
| 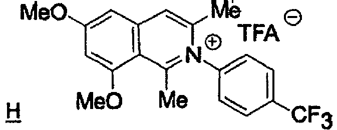 | 0.282 | 324 |
| 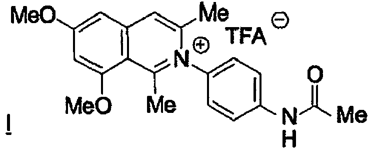 | 0.211 | > 841 |
| Chloroquin | 0.119 | n.d. |

Es wird deutlich, daß besonders Verbindung mit *para*-ständiger Acylamidogruppe, wie z.B. **I,** eine ausgeprägte Aktivität gegen den K1-Stamm von *P. falciparum* zeigen ohne nachweisbare Cytotoxizität. Substanz I dient dabei als bevorzugte Leitsubstanz, die sich auch zu einer weiteren chemischen Derivatisierung eignet.

### 4. Wachstumshemmende Wirkung (exemplarisch für A (siehe Beispiel 1) auf Gram-positive Bakterien der Gattung Staphylococcus und Hefepilze der Spezies Candida albicans

Anwendung: a) Therapie und Prävention von Infektionen durch Gram-positive Bakterien und pathogene Hefen in der Human- und Veterinärmedizin; b) Konservierung von Lebensmitteln, Arzneimitteln und Kosmetika durch Minderung der Keimlast und/ oder Verhinderung der Besiedlung durch Gram-positive Bakterien und Hefen.

### Untersuchung des inhibitorischen Effektes durch Bestimmung der minimalen Hemmkonzentration (MHK) im Mikrobouillon-Dilutionsverfahren

Die MHK (minimale Hemmkonzentration) ist die niedrigste Konzentration einer antibiotischen Substanz (in µM oder µg/ml), die das Keimwachstum unter Versuchsbedingungen gerade noch hemmt. Bei der Methode wird aus einer Substanzstammlösung eine Verdünnungsreihe mit abfallenden Wirkstoffkonzentrationen in 96-Loch-Mikrotiterplatten hergestellt. Die wirkstoffhaltigen Mikrotiterplatten-Vertiefungen werden mit einer definierten Menge des zu testenden Keimes beimpft und bebrütet. Die Konzentration der Substanz in der Vertiefung, in der optisch keine Trübung des Mediums durch ein Wachstum der Keime mehr nachweisbar ist, wird als die MHK angegeben.

### Technik

Die Teststämme wurden in Luria-Bertani-Medium angeimpft und über Nacht im Schüttelinkubator bei 37 °C bis zur stationären Wachstumsphase angezogen. Am folgenden Tag wurde die Kultur mit frischer Müller-Hinton-Bouillon (MH-Medium), die 5% NaCl (w/v) enthält, 1:100 verdünnt und bis zur logarithmischen Wachstumsphase erneut bei 37 °C inkubiert. Die optische Dichte der Bakteriensuspension wurde photometrisch bei 550 nm gemessen und eine Suspension von 2 x 10⁵ colony forming units / ml (CFU/ml) eingestellt. Inzwischen wurde eine geometrische Verdünnungsreihe der Testsubstanz in MH-Medium hergestellt. Von jeder Verdünnung wurden 100 µl in die entsprechenden Vertiefungen einer 96-Loch-Polystyren-Flachboden-Mikrotiterplatte pipettiert. Die Platten wurden mit 100 µl des vorbereiteten Inokulums beimpft und 18 Stunden bei 37 °C im Brutschrank bebrütet. Am nächsten Tag wurde die optische Dichte der bakteriellen Kulturen in den Vertiefungen mit einem ELISA-Reader bei einer Wellenlänge von 550 nm im Vergleich zum Leerwert (= unbeimpftes MH-Medium) bestimmt. Als MHK wurde die Konzentration in der Vertiefung angegeben, in der als letzte kein Wachstum der Keime mehr nachweisbar war. Eine niedrige MHK zeigt eine hohe inhibitorische Wirksamkeit einer Substanz an, während eine hohe Konzentration auf einen geringen Effekt schließen läßt.

### Ergebnisse

Die MHK z.B. von A wurden für folgende Keime und Stämme getestet:
*Staphylococcus aureus* 325 : Biofilm-positives klinisches Isolat aus Blutkultur, Wildtyp
*Staphylococcus aureus* NCTC 8325: Biofilm-negativer Referenzstamm,Genom publiziert
*Staphylococcus epidermidis* RP62A: Biofilm-positiver, multiresistenter Referenzstamm
*Escherichia coli* 536: Harnwegsisolat, Referenzstamm
*Pseudomonas aeruginosa:* Umweltisolat
*Candida albicans* 5314: klinisches Isolat

**Tabelle 4: Minimale Hemmkonzentrationen von A gegen verschiedene Infektionserreger**

| **Stamm** | **Minimale Hemmkonzentration (µM)** |
|---|---|
| *Staphylococcus aureus 325* | 0.63 |
| *Staphylococcus aureus* NCTC 8325 | 5.00 |
| *Staphylococcus epidermidis* RP62A | 0.63 |
| *Escherichia coli* 536 | 20.00 |
| *Pseudomonas aeruginosa* | >160 |
| *Candida albicans* 5314 | 1.25 |

### Bestimmung der Cytotoxizität durch den Alamar-Blue^{®}-Assay am Beispiel der Substanz A

Die Cytotoxizität von A wurde mittels eines Alamar-Blue^{®}-Assays bestimmt. Zuerst wurde eine geometrische Verdünnungsreihe von A im Zellmedium (293 Nierenepithelzellen; ohne Phenolrot) mit 1 % DMSO hergestellt und in 96-Loch-Polystyrol-Flachboden-Mikrotiterplatte vorgelegt. Die Zellen wurden trypsinisiert und einmal gewaschen. Für den Test wurde eine Zellsuspension 10⁵ Zellen/ml vorbereitet und 20 µl davon in die Vertiefungen pipettiert. Das Endvolumen betrug 200 µl. Nach 24h Inkubation bei 37 °C und 5 % CO₂ wurde in jede Vertiefung 20 µl Alamar Blue (Trinova Biochem, Gießen, Deutschland) zugefügt und die Mikrotiterplatte weiter inkubiert. Die Vertiefungen, die das Medium und Substanz, sowie die, die nur Zellsuspension (ohne Substanz) enthielten, dienten als negativ bzw. positiv Kontrolle. Die optische Dichte wurde nach 24h und 48h mit einem ELISA-Reader bei den Wellenlängen 550 nm und 630 nm gemessen. Der IC₅₀ Wert für A wurde nach linearer Interpolation (W. Huber, J. C. Koella Acta Tropica 1993, 55, 257-261) berechnet.

**Tabelle 5: Cytotoxizät von A und dessen therapeutischer Index gegen die beiden Biofilmbildenden Staphylokokken-Stämme sowie gegen C. albicans**

| | | | |
|---|---|---|---|
| 293 Nierenepithelzellen | *Staphylococcus aureus* 325 | *Staphylococcus epidermidis* RP62A | *Candida albicans* 5314 |
| 42.44 µM | 67 | 67 | 34 |

Wie aus Tabelle 2 ersichtlich ist, hat A eine hervorragende wachstumshemmende Wirkung gegen Gram-positive Infektionserreger wie Staphylokokken sowie gegen Hefepilze der Spezies *Candida albicans,* wobei dieser Effekt besonders deutlich gegen die beiden Biofilmbildenden Staphylokokken-Stämme ausgeprägt ist. Ebenfalls zeigen diese Stämme einen guten therapeutischen Index. Die Substanz hat eine geringere Wirksamkeit gegen Enterobakterien wie *E. coli* und ist ganz unwirksam gegen Pseudomonaden.

### 5. Hemmung der Biofilmbildung durch pathogene Staphylokokken (exemplarisch für A und S. epidermidis RP62A)

Anwendung: a) Therapie und Prävention von Fremdkörper-assoziierten Infektionen durch Biofilm-bildende Staphylokokken in der Human- und Veterinärmedizin; b) Verhinderung des Entstehens eines Staphylokokken-Biofilmes auf Kunststoff- und Metalloberflächen

### Bestimmung der Biofilmbildung von Staphylokokken auf Polystyren-Oberflächen (Biofilmtest)

### Technik

Zur Bestimmung der Wirkung einer Testsubstanz auf die Biofilmbildung von Staphylokokken wird der Biofilm-Referenzstamm *S*. *epidermidis* RP62A in Trypticase-Soy-Broth (TSB) mit der üblichen 0.25 % (w/v) Glucosekonzentration (Standard-TSB) angeimpft und über Nacht bei 37 °C im Schüttelinkubator bis zur stationären Wachstumsphase angezogen. Am nächsten Tag werden die Kulturen mit frischem TSB 1:100 verdünnt und 100 µl dieser Keimsuspension in die Vertiefungen einer 96-Loch-Polystyrol-Gewebekulturplatte mit flachem Boden (Greiner, Nürtingen, Deutschland) pipettiert. In der Zwischenzeit wird ebenfalls in TSB eine geometrische Verdünnungsreihe des Testsubstanz hergestellt und ebenfalls je 100 µl dieser Verdünnungen zu den Keimsuspensionen hinzugefügt. Die Platten werden bei 37 °C im Inkubator 18 Stunden lang bei 37 °C bebrütet. Am nächsten Tag wird zunächst das Wachstum der Kulturen durch Bestimmung der optischen Dichte bei 550 nm mittels eines ELISA-Readers geprüft (s. auch unter MHK-Bestimmung) und dokumentiert. Anschließend werden die Kulturen aus den Vertiefungen vorsichtig ausgegossen und die Platten dreimal mit Phosphatgepufferter Kochsalzlösung (PBS) gewaschen, um nicht-haftende Bakterien zu entfernen. Die Platten werden danach auf einem Heizblock bei ca 60 °C getrocknet, wobei anhaftenden Bakterien hitzefixiert werden. Zum Anfärben des Biofilms werden dann in die Vertiefungen der Platten je 100 µl einer gesättigten wäßrigen Kristallviolett-Lösung pipettiert, die nach 5 Minuten wieder entfernt wird. Überschüssiger Farbstoff wird anschließend unter reichlich fließendem Wasser abgewaschen. Nach dem Trocknen wird die Dichte des adhärenten Biofilms mit einem ELISA-Reader bei einer Wellenlänge von 490 nm bestimmt.

### Ergebnisse

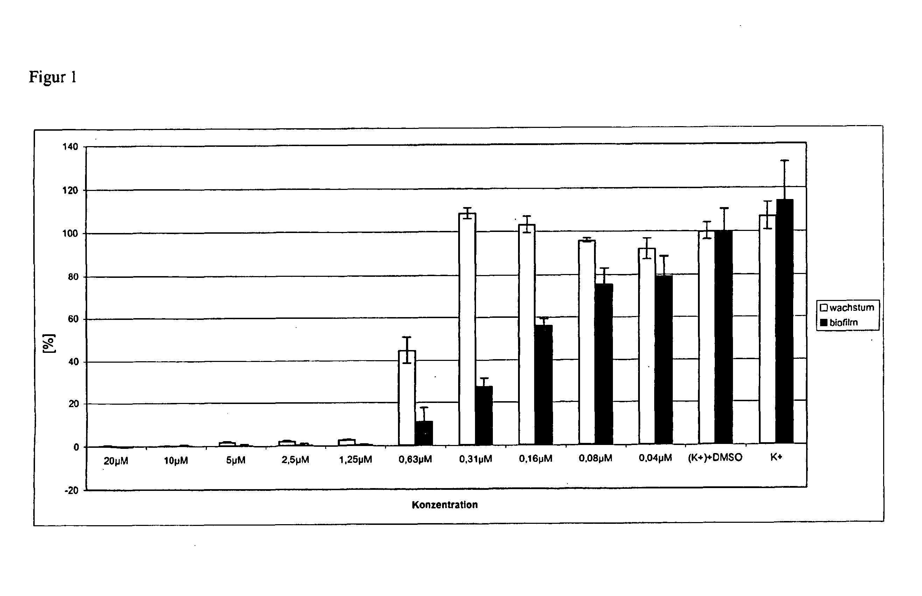

Wie aus Figur 1 ersichtlich ist, hemmt A die Biofilmbildung des Referenzstammes *S. epidermidis* RP62A hervorragend. Bei einer Wirkstoffkonzentration von 0.63 µM, die exakt der MHK der Substanz bei diesem Stamm entspricht, läßt sich eine ca. 90 %ige Verminderung der Biofilmbildung im Vergleich zur Kontrolle ohne die Substanz nachweisen (Abb. 1). Diese Wirkung nimmt dosisabhängig ab. Interessanterweise ist der Biofilm-hemmende Effekt auch noch deutlich bei solchen Konzentrationen nachweisbar, die offensichtlich bereits zu gering sind, um das Wachstum der Bakterien zu beeinflussen. Damit hat die Substanz, zusätzlich zum oben beschriebenen Wachstums-inhibierenden Effekt, eine davon unabhängige Anti-Biofilmwirkung auf Staphylokokken.

Figur 1 zeigt die Wirkung unterschiedlicher Konzentrationen von A auf Wachstum und Biofilmbildung von *S. epidermidis* RP62A.

## Patentansprüche

1. Antiinfektive sowie Biofilm-hemmende Verbindungen der allgemeinen Formeln 1 bis 2 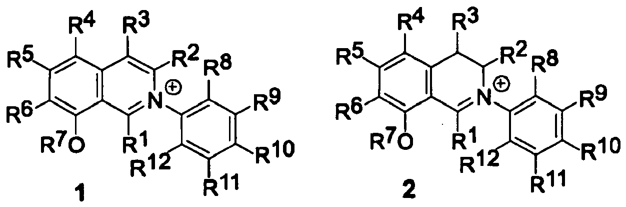 worin R³ H ist, R¹, R², R⁴ bis R⁶ und R⁸ bis R¹² unabhängig voneinander entweder H, ein unsubstituiertes, monosubstituiertes oder polysubstituiertes C₁-C₁₈-Alkyl, wobei das Alkyl gerade, verzweigt oder cyclisch sein kann, Alkenyl, ein unsubstituierter, monosubstituierter oder polysubstituierter Aryl- oder Heteroarylrest, eine unsubstituierte, monosubstituierte oder polysubstituierte Benzylgruppe, eine Acylgruppe, wie z.B. Formyl, Acetyl, Trichloracetyl, Fumaryl, Maleyl, Succinyl, Benzoyl, oder eine verzweigte oder Heteroatom- oder arylsubstituierte Acylgruppe, ein Alkoxysubstituent, wie z.B. -OMe, -OEt, -O*n*Pr, -iPr, -O*n*Bu, -O*i*Bu, *-OsecBu,* -O*t*Bu, dessen Alkylgruppe verzweigt, unverzweigt oder cyclisch ist, eine über ein Schwefelatom gebundene Alkylgruppe, wie z.B. -SMe, -SEt, oder eine Sulfonylgruppe, wie z. B. -SO₃H, -SO₂Me, -SO₂CF₃, -SO₂C₆H₄CH₃ oder SO₂C₆H₄CH₂Br, oder ein Stickstoffsubstituent, wie z.B. NH₂, -NHR, -NRR' (mit R, R' = Alkyl, Aryl usw.),-NC oder -NO₂, oder Fluor, Chlor, Brom, Jod, -CN ist,
R⁷ unabhängig voneinander entweder H, ein unsubstituiertes, monosubstituiertes oder polysubstituiertes C₁-C₁₈-Alkyl, wobei das Alkyl gerade, verzweigt oder cyclisch sein kann, ein monosubstituiertes oder polysubstituiertes, gerades, verzweigtes oder cyclisches C₁-C₁₈-Alkenyl oder eine Acylgruppe, wie z.B. Formyl, Acetyl, Trichloracetyl, Fumaryl, Maleyl, Succinyl, Benzoyl, verzweigte oder Heteroatom- oder arylsubstituierte Acylgruppen sein kann,
und R⁸ bis R¹² auch in der Art verknüpft sein können, dass dadurch ein unsubstituierter, monosubstituierter oder polysubstituierter Ring und Dimere von **1** entsteht, sowie pharmazeutisch verträgliche Salze oder Solvate dieser Verbindung, mit der Maßgabe, dass Formel **1-2** nicht die folgenden Formeln 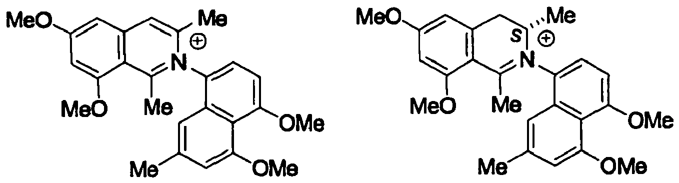 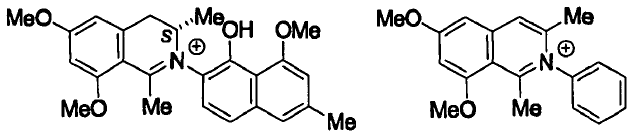 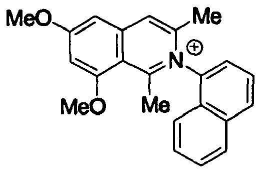 bedeuten, zur Behandlung und/oder Prävention von Erkrankungen.

2. Antiinfektive sowie Biofilm-hemmende Verbindung nach Anspruch 1 der Formel A (=1a) 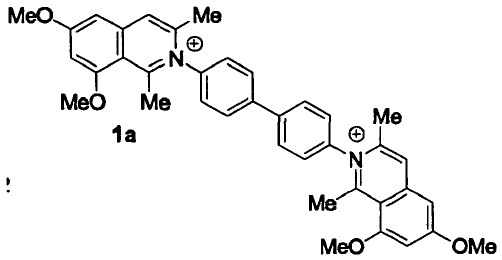 sowie pharmazeutisch verträgliche Salze oder Solvate dieser Verbindung, zur Behandlung und/oder Prävention von Erkrankungen.

3. Antiinfektive sowie Biofilm-hemmende Verbindung nach Anspruch 1 der Formel 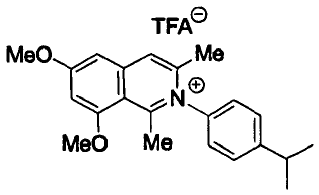 zur Behandlung und/oder Prävention von Erkrankungen.

4. Antiinfektive sowie Biofilm-hemmende Verbindung nach Anspruch 1 der Formel 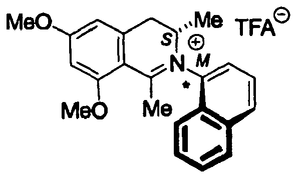 zur Behandlung und/oder Prävention von Erkrankungen.

5. Verbindung nach Anspruch 1 der Formel 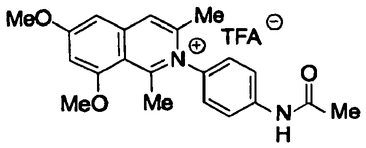 zur Behandlung und/oder Prävention von Erkrankungen.

6. Verfahren zur Herstellung einer antiinfektiven sowie Biofilm-hemmende Verbindung nach Anspruch 4, die Umsetzung von 2,6-Lutidin, 1-Naphthylboronsäure, Kupfer(II)acetat und Myristinsäure mit einem primären Amin.

7. Pharmazeutische Zusammensetzung, umfassend eine therapeutisch wirksame Menge einer antiinfektiven sowie Biofilm-hemmende Verbindung nach einem der Ansprüche 1 bis 5, gegebenenfalls zusammen mit geeigneten Zusatz- oder Hilfsstoffen.

8. Pharmazeutische Zusammensetzung nach Anspruch 7, **dadurch gekennzeichnet, dass** die Verbindung in Form einer Depotsubstanz oder als Vorläufer zusammen mit einer geeigneten, pharmazeutisch verträglichen Verdünnungslösung oder Trägersubstanz vorliegt.

9. Pharmazeutische Zusammensetzung nach Anspruch 7 oder 8, **dadurch gekennzeichnet, dass** sie als Oberflächenbeschichtung, als Zusatz von Werkstoffen oder Spüllösungen vorliegt.

10. Pharmazeutische Zusammensetzung nach einem der Ansprüche 7 bis 9 in Form von Tabletten, Dragees, Kapseln, Tropflösungen, Suppositorien, Injektions- oder Infusionszubereitungen zur peroralen, rektalen oder parenteralen Anwendung.

11. Verwendung einer antiinfektiven sowie Biofilm-hemmende Verbindung nach einem der Ansprüche 1 bis 5 oder einer pharmazeutischen Zusammensetzung nach einem der Ansprüche 7 bis 10 zur Herstellung eines Medikaments zur Verhinderung der Biofilmbildung auf Oberflächen sowie zur Behandlung und/oder Vorbeugung von Infektionskrankheiten.

12. Verwendung nach Anspruch 11 zur Behandlung und/oder der Vorbeugung von Malaria oder der Verhinderung der Biofilmbildung durch S. *epidermidis.*

## Claims

1. Anti-infective as well as biofilm-inhibiting compounds of the general formulae 1 to 2 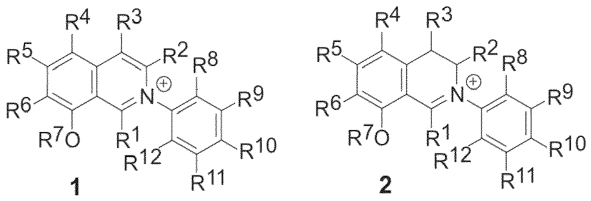 Wherein R³ is H, R¹, R², R⁴ to R⁶ and R⁸ to R¹² independently is either H, a non-substituted, monosubstituted or polysubstituted C₁-C₁₈-alkyl, wherein the alkyl can be straight, branched or cyclic, alkenyl, a non-substituted, monosubstituted or polysubstituted aryl or heteroaryl residue, a non-substituted, monosubstituted or polysubstituted benzyl group, an acyl group, such as, for example, formyl, acetyl, trichloroacetyl, fumaryl, maleyl, succinyl, benzoyl, or a branched or heteroatom- or aryl-substituted acyl group, an alkoxy substituent, such as, for example, -OMe, -OEt, - OnPr, -*i*Pr, -O*n*Bu, -O*i*Bu, -O*sec*Bu, -O*t*Bu, the alkyl group thereof is branched, non-branched or cyclic, an alkyl group bound through a sulfur atom, such as, for example,-SMe, -SEt, or a sulfonyl group, such as, for example, -SO₃H, -SO₂Me, -SO₂CF₃,-SO₂C₆H₄CH₃ or SO₂C₆H₄CH₂Br, or a nitrogen substituent, such as, for example, -NH₂, -NHR, -NRR' (with R, R' = alkyl, aryl, etc.), -NC or -NO₂, or fluoro, chloro, bromo, iodo, -CN,
R⁷ independently can be either H, a non-substituted, monosubstituted or polysubstituted C₁-C₁₈-Alkyl, wherein the alkyl can be straight, branched or cyclic, a monosubstituted or polysubstituted, straight, branched or cyclic C₁-C₁₈-alkenyl or can be an acyl group, such as, for example, formyl, acetyl, trichloroacetyl, fumaryl, maleyl, succinyl, benzoyl, branched or heteroatom- or aryl-substituted acyl groups,
and R⁸ to R¹² can also be bonded in a manner that thereby a non-substituted, monosubstituted or polysubstituted ring and dimers of **1** is generated, as well as pharmaceutically acceptable salts or solvates of this compound, with the provisio that formulae **1-2** do not mean the following formulae 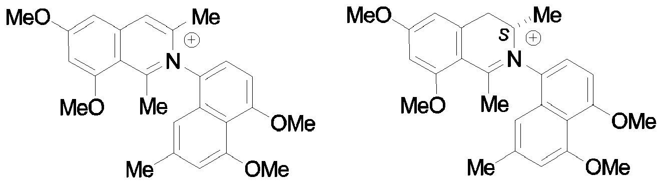 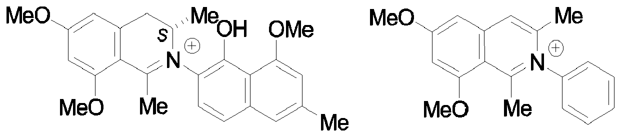 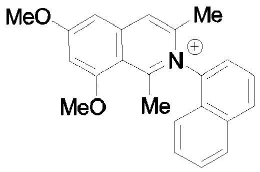 for a treatment and/or the prevention of diseases.

2. Anti-infective as well as biofilm-inhibiting compound according to claim 1 of the
formula A (=1a) 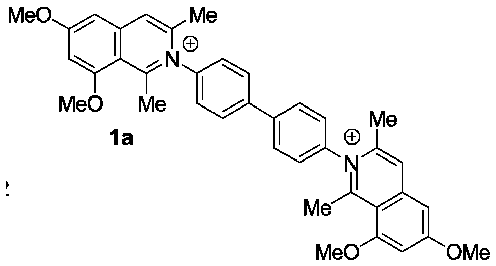 as well as pharmaceutically acceptable salts or solvates of this compound for a treatment and/or the prevention of diseases.

3. Anti-infective as well as biofilm-inhibiting compound according to claim 1 of the
formula 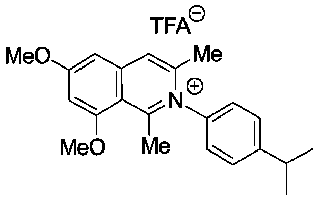 for a treatment and/or the prevention of diseases.

4. Anti-infective as well as biofilm-inhibiting compound according to claim 1 of the
formula 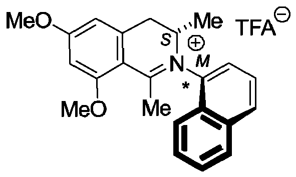 for a treatment and/or the prevention of diseases.

5. The compound according to claim 1 of the formula 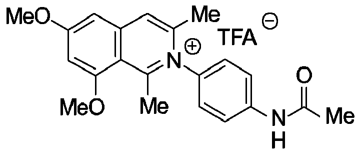 for a treatment and/or the prevention of diseases.

6. The method for producing a anti-infective as well as biofilm-inhibiting compound according to claim 4, the reaction of 2,6-lutidine, 1-naphthyl boric acid, copper(II)acetate, and myristic acid with a primary amine.

7. Pharmaceutical composition comprising a therapeutically effective amount of an anti-infective as well as biofilm-inhibiting compound according to any of claims 1 to 5, optionally together with suitable additives or excipients.

8. Pharmaceutical composition according to claim 7, **characterized in that** the compound is present in the form of a depot substance, or as a precursor, together with a suitable pharmaceutically acceptable diluent or carrier substance.

9. Pharmaceutical composition according to claim 7 or 8, **characterized in that** it is present as surface coating, as additive of materials or solutions for rinsing.

10. Pharmaceutical composition according to any of claims 7 to 9 in the form of tablets, dragées, capsules, drop-solutions, suppositories, preparations for injection or infusion, for a peroral, rectal or parenteral use.

11. Use of an anti-infective as well as biofilm-inhibiting compound according to any of claims 1 to 5 or a pharmaceutical composition according to any of claims 7 to 10 for the production of a medicament for the prevention of the formation of biofilms on surfaces as well as for a treatment and/or the prophylaxis of infectious diseases.

12. Use according to claim 11 for a treatment and/or the prophylaxis of malaria, or the prevention of the formation of biofilms by *S. epidermidis.*

## Revendications

1. Composés anti-infectieux ainsi qu'inhibiteurs de biofilm correspondant aux formules générales 1 à 2 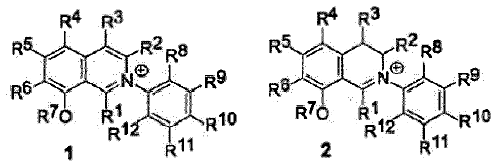 où R³ est un atome d'hydrogène, R¹, R², R⁴ à R⁶ et R⁸ à R¹² sont indépendamment les uns des autres soit un atome d'hydrogène, un alkyle en C₁-C₁₈ non substitué, monosubstitué ou polysubstitué, l'alkyle pouvant être linéaire, ramifié ou cyclique, un alcényle, un résidu aryle ou hétéroaryle non substitué, monosubstitué ou polysubstitué, un groupe benzyle non substitué, monosubstitué ou polysubstitué, un groupe acyle, comme par exemple formyle, acétyle, trichloracétyle, fumaryle, maléyle, succinyle, benzoyle, ou un groupe acyle ramifié ou substitué par un hétéroatome ou un groupe aryle, un substituant alcoxy, comme par exemple -OMe, -OEt, O*n*Pr, -iPr, -O*n*Bu,-OiBu, -OsecBu, -O*t*Bu, dont le groupe alkyle est ramifié, non ramifié ou cyclique, un groupe alkyle lié par l'intermédiaire d'un atome de soufre, comme par exemple -SMe, -SEt ou un groupe sulfonyle, comme par exemple - SO₃H, -SO₂Me, -SO₂CF₃, -SO₂C₆H₄CH₃ ou SO₂C₆H₄CH₂Br ou un substituant azote, comme par exemple -NH₂, -NHR, -NRR' (avec R, R' = alkyle, aryle, etc.), -NC ou -NO₂, ou du fluor, du chlore, du brome, de l'iode, -CN,
R⁷ peut être indépendamment des autres soit un atome d'hydrogène, un alkyle en C₁-C₁₈ non substitué, monosubstitué ou polysubstitué, l'alkyle pouvant être linéaire, ramifié ou cyclique, un alcényle en C₁-C₁₈ monosubstitué ou polysubstitué, linéaire, ramifié ou cyclique ou un groupe acyle, comme par exemple formyle, acétyle, trichloracétyle, fumaryle, maléyle, succinyle, benzoyle, des groupes acyle ramifiés ou substitués par un hétéroatome ou un groupe aryle,
et R⁸ à R¹² peuvent également être liés de sorte qu'il en résulte un cycle non substitué, monosubstitué ou polysubstitué et des dimères de 1, ainsi que des sels ou des solvates pharmaceutiquement compatibles de ce composé, sous réserve que les formules 1 à 2 ne correspondent pas aux formules ci-dessous 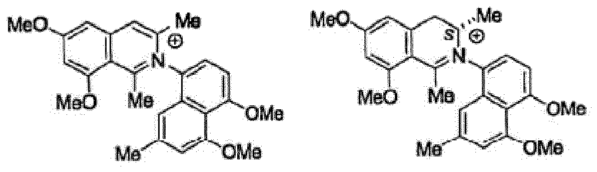 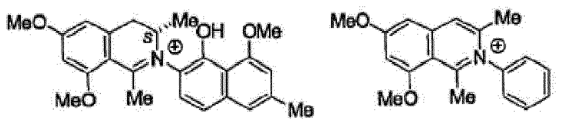 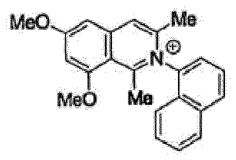 destinés au traitement et/ou à la prévention de maladies.

2. Composé anti-infectieux ainsi qu'inhibiteur de biofilm selon la revendication 1 de formule A (= la) 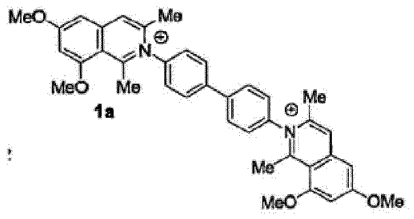 ainsi que des sels ou des solvates pharmaceutiquement compatibles de ce composé, pour le traitement et/ou la prévention de maladies.

3. Composé anti-infectieux ainsi qu'inhibiteur de biofilm selon la revendication 1 de formule 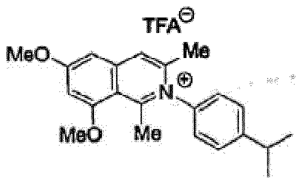 pour le traitement et/ou la prévention de maladies.

4. Composé anti-infectieux ainsi qu'inhibiteur de biofilm selon la revendication 1 de formule 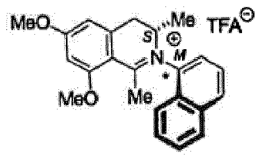 pour le traitement et/ou la prévention de maladies.

5. Composé selon la revendication 1 de formule 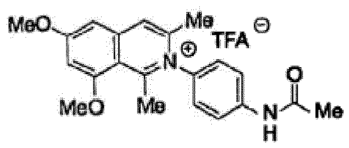 pour le traitement et/ou la prévention de maladies.

6. Procédé de préparation d'un composé anti-infectieux ainsi qu'inhibiteur de biofilm selon la revendication 4, comprenant la réaction de 2,6-lutidine, d'acide 1-naphtylboronique, d'acétate cuivrique et d'acide myristique avec une amine primaire.

7. Composition pharmaceutique, comprenant une quantité thérapeutiquement efficace d'un composé anti-infectieux ainsi qu'inhibiteur de biofilm selon l'une quelconque des revendications 1 à 5, éventuellement en commun avec des additifs ou des adjuvants appropriés.

8. Composition pharmaceutique selon la revendication 7, **caractérisé en ce que** le composé est présent sous la forme d'une substance de dépôt ou en tant que précurseur en commun avec une solution de dilution ou une substance de support pharmaceutiquement compatible appropriée.

9. Composition pharmaceutique selon la revendication 7 ou 8, **caractérisé en ce qu'**elle est présente en tant que revêtement de surface, sous forme d'additif de matériaux ou de solutions de rinçage.

10. Composition pharmaceutique selon l'une quelconque des revendications 7 à 9 sous forme de comprimés, pastilles, gélules, solutions en gouttes, suppositoires, préparations pour injection ou pour perfusion destinées à une utilisation per-orale, rectale ou parentérale.

11. Utilisation d'un composé anti-infectieux ainsi qu'inhibiteur de biofilm selon l'une quelconque des revendications 1 à 5 ou d'une composition pharmaceutique selon l'une quelconque des revendications 7 à 10 pour la préparation d'un médicament destiné à empêcher la formation de biofilm sur des surfaces ainsi que pour le traitement et/ou la prévention de maladies infectieuses.

12. Utilisation selon la revendication 11 pour le traitement et/ou la prévention de la malaria ou pour empêcher la formation d'un biofilm par S. *epidermidis.*
